# FASCICULE DE BREVET EUROPEEN

(11) **EP 2 978 970 B1**
(45) Date de publication et mention de la délivrance du brevet: **22.02.2017**
(21) Numéro de dépôt: 14713125.4
(22) Date de dépôt: 27.03.2014
(51) Int. Cl.: F04B 43/09

(54) **POMPE D'INJECTION D'UN FLUIDE, ET NOTAMMENT MICROPOMPE UTILISABLE POUR DÉLIVRER UNE DOSE DETERMINÉE**
FLÜSSIGKEITSINJEKTIONSPUMPE, INSBESONDERE MIKROPUMPE, ZUR ABGABE EINER BESTIMMTEN ABGEMESSENEN DOSIS
FLUID INJECTION PUMP, NOTABLY MICROPUMP, THAT CAN BE USED FOR DELIVERING A DETERMINED METERED DOSE

(30) Priorité: 28.03.2013 FR 1352850
(43) Date de publication de la demande: 03.02.2016
(73) Titulaire: Commissariat à l'Énergie Atomique et aux Énergies Alternatives, 75015 Paris (FR)
(72) Inventeur: DELEVOYE, Elisabeth, F-38120 Saint Egrève (FR); NIKOLOVSKI, Jean-Pierre, 13620 Carry le Rouet (FR)
(74) Mandataire: Brevalex
(86) Numéro de dépôt international: PCT/EP2014/056226
(87) Numéro de publication internationale: WO 2014/154840

(56) Documents cités:
- WO-A1-2011/113938
- JP-A- H05 195 958
- JP-A- 2001 271 757
- JP-A- 2008 088 904

## Description

L'invention est une pompe d'injection d'un fluide, et notamment une micropompe utilisable pour délivrer une dose déterminée.

Cette pompe fait appel à un résonateur comprenant un transducteur piézoélectrique et un solide solidaire du transducteur et qui se déforme quand le transducteur est excité. Elle comprend encore un manchon, creusé d'un conduit central dans lequel le fluide s'écoule. Le manchon est en contact avec le solide. Quand le solide se déforme de façon oscillante, les battements qu'il communique au manchon suscitent des mouvements du fluide qui provoquent sa circulation le long du manchon. Cette circulation peut être due soit à des modes particuliers de déformation, qui la favorisent dans un sens déterminé, soit à une différence de pression existant entre les extrémités du manchon, par exemple entre un réservoir du fluide en surpression en amont de la pompe et un point d'injection à pression ambiante en aval, mais qui est trop faible pour établir cette circulation au repos. JP H05 195958 divulgue l'état de la technique le plus proche.

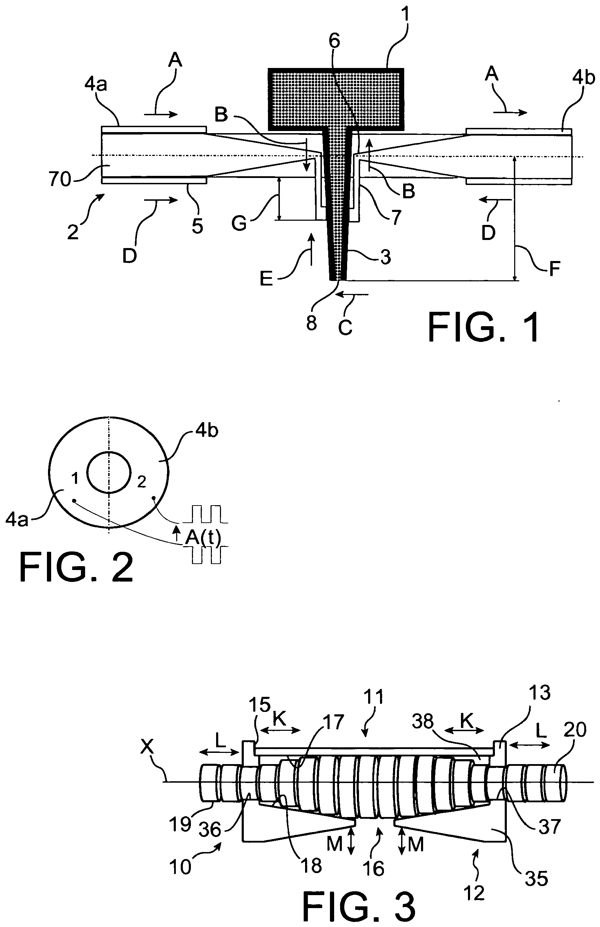

On va décrire une telle pompe d'injection (qui ne fait pas partie de l'invention) au moyen des figures 1 et 2. La pompe intègre un réservoir 1 contenant un fluide couplé à un résonateur 2 par un manchon 3 creux formant un tube flexible. Le résonateur 2 est annulaire et entoure le manchon 3. Il comprend un anneau 70 en métal qui possède sur sa périphérie des transducteurs piézoélectriques qui le déforment quand de l'énergie électrique leur est appliquée. Ces transducteurs comprennent tous une électrode externe commandée par une alimentation extérieure, une électrode interne et une couche en matière piézoélectrique qui se déforme selon le champ électrique que lui appliquent les électrodes. Les électrodes sont fixées sur les deux faces de la couche piézoélectrique, l'électrode interne est aussi fixée à l'anneau 70, et l'électrode externe donne à l'extérieur du résonateur 2. Les déformations de la couche piézoélectrique sont donc transmises ainsi à l'anneau 70. Le résonateur 2 est relié au réservoir 1 par l'intermédiaire d'au moins le manchon 3. Les déformations que les transducteurs appliquent à l'anneau 70 du résonateur 2 sont en direction radiale, vers l'intérieur ou l'extérieur selon le signe de charges électriques appliquées à leurs électrodes. Les tensions appliquées étant alternatives, ces déformations oscillent autour du point de repos. Les transducteurs comprennent une paire de transducteurs semi-circulaires 4a et 4b (Figure 2) fixée à la face supérieure de l'anneau 70, et qui peuvent être commandés par des tensions alternatives (A) en opposition de phase. Les déformations qu'ils font subir à l'anneau 70 sont donc de sens opposés sur des rayons opposés. L'anneau 70 se déforme dans l'épaisseur selon les contraintes mécaniques induites par les transducteurs 4a et 4b, et sa rigidité fait que ces déformations induisent à la partie centrale 6 des déflexions verticales opposées (B) des secteurs de l'anneau 70, qui sont respectivement associés aux transducteurs 4a et 4b, résultant en une flexion latérale (C) du manchon 3, qui le fait osciller d'un côté puis de l'autre à chaque inversion de sens des tensions (A). Cette oscillation de flexion du manchon cisaille le fluide, provoquant l'écoulement du contenu du réservoir 1. Un second transducteur ayant une électrode externe 5 et une électrode interne à la masse est collé sous l'anneau 70, en regard des transducteurs 4a à 4b, et soumet l'anneau 70 à des déformations axisymétriques radiales (D). L'électrode externe du transducteur 5 peut ne comporter qu'un seul quadrant ou bien deux quadrants comme le transducteur 4 (pour donner aux transducteurs 4 et 5 des fonctions électriques interchangeables) mais dans ce cas les tensions électriques sont appliquées en phase, contrairement aux tensions (A) qui sont en opposition de phase. De la même façon qu'avec les transducteurs 4a et 4b, ces déformations correspondantes engendrent une composante verticale de déformation du centre de l'anneau piézoélectrique, dont l'orientation est uniforme sur sa circonférence, de sorte qu'elles induisent un mouvement vertical (E) du manchon 3. Ici encore, l'application d'une tension alternative produit un mouvement oscillatoire. Ce mode de vibration du manchon dans une direction axiale, qu'on appellera aussi mode haut-parleur puisqu'il se traduit par un battement de la face inférieure du réservoir 1 à laquelle le manchon 3 est relié, peut aussi faciliter l'écoulement du fluide hors du réservoir. Il faut toutefois souligner qu'une configuration particulièrement favorable est obtenue en cumulant les deux modes d'excitation, la fréquence d'oscillation axiale ou verticale du manchon 3 (d'après le mouvement E) étant deux fois celle d'oscillation de flexion du manchon 3 (d'après le mouvement C), puisqu'alors l'écoulement est beaucoup facilité, et le débit hors du réservoir 1 peut être soit plus important, soit obtenu avec des tensions appliquées diminuées de plus d'un facteur 2.

L'anneau 70 présente une région centrale 6 amincie réalisant une amplification mécanique de la vibration. La région centrale 6 du résonateur 2 comprend une section tubulaire 7, en contact avec le manchon 3 effilé vers son extrémité libre du manchon, où se trouve une buse d'éjection 8. Les mouvements du centre de l'anneau 70 sont donc transmis au manchon 3 par la section tubulaire 7, qui convertit le mouvement surtout vertical B en mouvement surtout horizontal C par effet de levier. Cette disposition permet aussi de raccorder le résonateur 2 à un endroit plus effilé constituant une charge mécanique plus faible du manchon 3 et donc de le fléchir plus facilement, tout en transmettant des oscillations plus importantes que celles de la région centrale 6, encore grâce au bras de levier procuré par la section tubulaire 7 ; l'amincissement de la région centrale 6 permet lui-même d'augmenter ces oscillations grâce à la diminution de sa rigidité.

Parmi les avantages de cette pompe d'injection particulière, on mentionnera qu'elle permet de délivrer des microdébits (typiquement inférieurs à 1 microlitre à l'heure) avec une très grande précision et des quantités de fluide tout aussi précises, même extrêmement petites, typiquement inférieures à quelques microlitres. Son encombrement est notamment très réduit, ce qui permet de l'utiliser facilement dans des applications biologiques ou chirurgicales. On peut par exemple citer le cas d'une pompe implantée dans l'oreille moyenne à la suite d'un abord chirurgical dénommé tympanotomie postérieure. La pompe peut être mise en action et arrêtée sans la moindre difficulté. Il existe d'autres types de pompes commandées par des résonateurs piézoélectriques ou d'autres générateurs de vibration, mais qui sont en général d'un encombrement plus grand ou dont le débit de fuite est supérieur au débit souhaité ou qui ne permettent pas de doser le fluide délivré selon des doses aussi petites ou avec une précision insuffisante dans le dosage.

L'invention présente a pour objectif de renforcer les avantages des pompes d'injection analogues à celle des figures 1 et 2, et notamment de permettre une réduction supplémentaire de l'encombrement et une efficacité plus grande de l'énergie d'excitation du transducteur piézoélectrique, sans diminution de la précision du débit ou de la quantité de fluide injectée à chaque période de fonctionnement. De plus, l'invention permet d'obtenir un dispositif de forme aplatie, dont l'épaisseur est faible par rapport au diamètre dans lequel il s'inscrit, ce qui favorise la possibilité d'implantation dans un patient, en particulier en sous-cutané ou dans des emplacements exigus, par exemple dans une cavité creusée dans la mastoïde.

Sous une forme générale, l'invention est relative à une pompe d'injection d'un fluide, comprenant un résonateur et un manchon, le résonateur comprenant un transducteur piézoélectrique et un solide que le transducteur déforme, et le manchon était parcouru par le fluide pendant l'injection, et s'étendant selon un axe, le manchon étant apte à être déformé par le solide se déformant,
caractérisée en ce que
le résonateur comprend une surface d'appui, destinée à entrer en contact avec le manchon lorsque ce dernier est déformé par ledit solide,
et le transducteur s'étend parallèlement à l'axe du manchon.

La pompe peut également comprendre une des caractéristiques suivantes, prises isolément ou en combinaison :
- le solide comprend une première partie, annulaire, s'étendant selon une direction parallèle à l'axe du manchon, et apte à exercer une pression sur le manchon sous l'effet d'une deuxième partie, solidaire de la première partie, s'étendant selon une direction transversale, notamment perpendiculaire, à l'axe du manchon;
- le transducteur peut être plan, par exemple en forme de disque ou de secteur de disque, auquel cas la direction selon laquelle il s'étend, dite direction d'extension, correspond à une direction inscrite dans le plan. Dans ce cas, la première partie dudit solide s'étend selon une direction parallèle à ce plan ;
- le transducteur peut être formé par une surface légèrement incurvée, par exemple en forme de disque ou de secteur légèrement incurvé, auquel cas la direction selon laquelle il s'étend correspond à une direction reliant un point situé au centre du disque et un point situé à la périphérie du transducteur ;
- ladite deuxième partie forme une rehausse, qui s'étend à partir d'une zone périphérique de l'anneau formé par la première partie ;
- le solide comprend une première partie s'étendant selon une direction sensiblement parallèle à l'axe du manchon, et comprend une surface destinée à exercer une pression sur le manchon afin de le déformer ;
- sous l'effet de l'oscillation de la deuxième partie (par exemple une flexion), la première partie est adaptée pour subir une oscillation de façon à exercer une force perpendiculaire à l'axe du manchon ;
- le solide comporte une deuxième partie apte à entrer en contact avec le transducteur : elle peut le maintenir, et avantageusement appliquer une précontrainte sur le transducteur en le comprimant ;
- ladite deuxième partie du solide s'étend de la première partie du solide vers une bordure libre. Le contact entre le transducteur et la deuxième partie peut se situer à cette bordure, ce qui permet d'avoir un dispositif particulièrement compact. Le contact entre le transducteur et la deuxième partie peut se situer entre la première partie et la bordure. Plus la distance entre la bordure et le point de contact est élevée, plus l'effet de levier permettant d'appliquer une précontrainte sur le transducteur peut être amplifié ;
- la première partie et la deuxième partie du solide, ainsi que la surface d'appui délimitent une cavité, à travers laquelle s'étend le manchon. Ladite deuxième partie peut notamment comprendre des ouvertures, de telle sorte que le manchon puisse s'étendre de part et d'autre de cette cavité.

On passe ainsi d'une disposition où la pompe a une configuration généralement cylindrique (le résonateur entourant le manchon, dont l'axe est perpendiculaire au plan du solide annulaire déformable) à une configuration plane, où l'axe du manchon est parallèle à la direction principale d'extension du solide. Cette nouvelle configuration, dans laquelle la pompe s'étend le long du manchon, est moins encombrante. Elle est aussi susceptible d'être plus efficace, à cause du contact plus grand entre le manchon et le solide qui applique les oscillations, ce qui permet un meilleur couplage. Et la surface d'appui permet que les oscillations perpendiculaires au manchon ne le secouent pas seulement mais le compriment, ce qui devrait faciliter encore l'écoulement.

Selon diverses façons avantageuses de réaliser l'invention, la première partie du solide peut être une plaquette apte à être déformée en flexion, et ladite surface du solide est une face principale de la plaquette, c'est-à-dire une des faces perpendiculaires à la plus petite dimension (l'épaisseur de la plaquette), ce qui permet une bonne efficacité d'utilisation de l'énergie excitatrice du transducteur pour écouler le fluide. La plaquette peut en particulier consister en un disque.

La plaquette peut être munie d'une rehausse périphérique transversale, notamment perpendiculaire à ladite plaquette, formant alors une deuxième partie du solide. Le transducteur peut alors être encastré dans la rehausse, ce qui permet une transmission directe de l'énergie du transducteur au solide à travers l'interface d'encastrement, en l'absence de tout collage. La plaquette peut être annulaire et le transducteur peut être un disque ; la rehausse peut consister en des segments angulaires d'un anneau, les segments étant disjoints. L'encastrement du transducteur est alors facilité. La plaquette peut avoir une épaisseur constante, mais de préférence l'épaisseur diminue en direction de son centre.

Ainsi, la plaquette en forme d'anneau peut être amincie à une portion centrale, afin de l'assouplir et d'amplifier l'amplitude des oscillations à cet endroit, notamment les modes selon lesquelles les oscillations sont maximales au niveau de cette portion centrale. Cette plaquette peut avoir une forme de disque ou anneau plein, mais d'une façon générale, on préfère une forme annulaire, comportant une ouverture centrale. La section de cette plaquette peut ne pas être constante.

Ainsi, le solide, apte à être déformé par le transducteur, comporte une première partie en forme d'anneau s'étendant selon une direction parallèle au disque formé par le transducteur, entre sa périphérie et son centre. Le terme anneau désigne un disque plein ou une surface, notamment un disque, comportant une ouverture centrale.

Ce solide comporte en outre une deuxième partie, transversale à la première partie précédemment décrite. Elle peut alors former une rehausse, s'étendant de la première partie vers une bordure libre. La première partie s'étend notamment perpendiculairement à la deuxième partie.

La surface d'appui peut appartenir soit à une plaque indépendante, soit au transducteur lui-même, ce qui conduit à une simplification de la pompe.

Le manchon s'étend à travers une cavité délimitée par la première partie, la deuxième partie et la surface d'appui, cela par le moyen d'ouvertures ménagées dans la première partie, ces ouvertures pouvant être diamétralement opposées. Il peut avantageusement être muni d'embouts de branchement opposés. La pompe forme alors un équipement complètement autonome qu'on peut associer au reste d'un dispositif d'injection, en raccordant deux cathéters aux embouts. Le manchon peut aussi être conçu comme un réservoir de fluide intermédiaire dont la section d'écoulement est plus grande que celle des embouts, de manière à accumuler une certaine quantité du fluide à injecter : on constate alors que les conditions de l'écoulement, et en particulier son débit, ne dépendent pas de la pression à l'intérieur du réservoir amont de liquide, contrairement à ce qui pouvait être le cas dans les dispositifs antérieurs.

La pompe peut encore comprendre un bouchon ajusté avec serrage autour d'une bordure libre de la rehausse, afin à la fois de couvrir le transducteur pour le protéger. Cela permet également à la rehausse d'appliquer une force de compression sur le transducteur. Autrement dit, le transducteur est encastré dans la rehausse, et avantageusement selon une précontrainte radiale. Cela permet aussi d'instaurer une contraction de la rehausse et un effort d'encastrement du transducteur. Le bouchon est de préférence inséré en force au niveau de la bordure de la deuxième partie, de façon à générer ladite précontrainte. Toute autre méthode connue de l'homme de l'art visant à appliquer une précontrainte radiale en compression peut être employée.

La pompe d'injection peut faire partie d'un dispositif d'injection de fluide comprenant encore un réservoir du fluide, un premier cathéter reliant le réservoir au manchon, et un deuxième cathéter reliant le manchon au point d'injection du fluide, le manchon reliant alors les cathéters entre eux, le dispositif comprenant encore un excitateur du transducteur.

Par excitateur du transducteur, on entend des moyens pour déformer ce dernier en lui appliquant des signaux électriques. Il s'agit notamment d'électrodes disposées à la surface du transducteur, reliées à une ou plusieurs alimentations électriques.

On a vu qu'une propriété importante de telles pompes était l'absence de débit de fuite pendant les périodes d'inactivité, c'est-à-dire en dehors des périodes d'excitation du transducteur, du fait de l'action de forces de capillarité dans le conduit (manchon ou cathéter), exerçant une résistance à l'écoulement spontané du fluide. Il est toutefois possible, en cas de besoin, d'ajouter une valve d'ouverture à une pression déterminée du fluide, qu'on place dans ledit conduit. Cela permet notamment d'augmenter le diamètre du conduit, pour abaisser la pression nécessaire pour produire un écoulement. On réduit alors la consommation électrique du dispositif.

L'invention sera maintenant décrite plus en détail en liaison aux figures suivantes :
- la figure 1, déjà décrite, représente une pompe n'appartenant pas à l'invention ;
- la figure 2 est une vue de dessus du résonateur de la pompe de la figure 1 ;
- la figure 3 illustre une réalisation de l'invention ;
- la figure 4 illustre la cavité résonante d'une variante de réalisation ;
- la figure 5 illustre complètement cette variante de réalisation ;
- et la figure 6 illustre un dispositif de pompage complet.

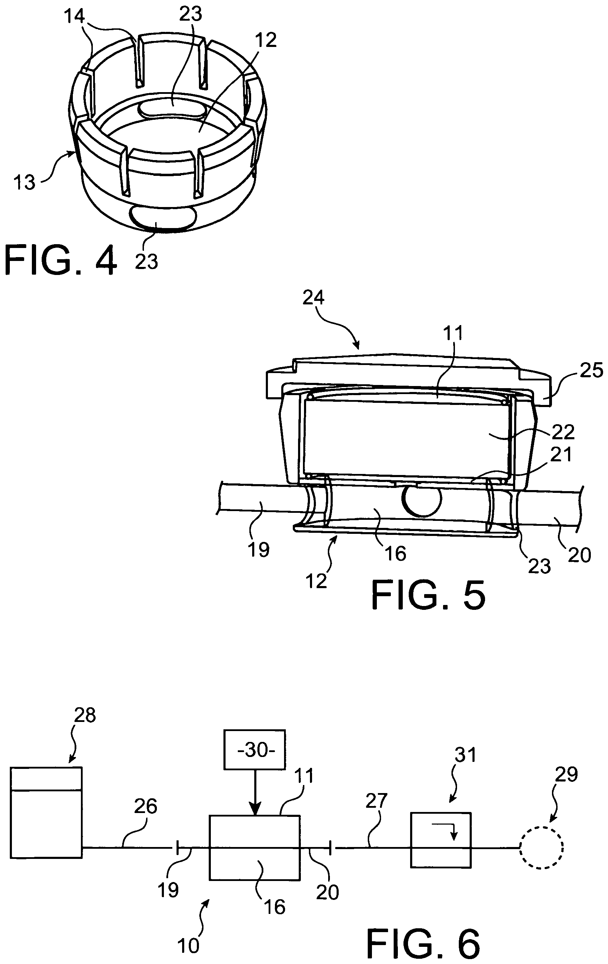

Une réalisation de l'invention est décrite d'abord à la figure 3. La pompe est composée essentiellement d'un résonateur 10 et d'un manchon 16 d'axe X. Le résonateur 10 comprend un transducteur 11 en forme de disque plat, et un solide 12, excité par le transducteur 11, comprenant une première partie 35 en forme de disque parallèle ou essentiellement parallèle au transducteur 11, s'amincissant toutefois de la périphérie au centre et étant de préférence évidé dans sa partie centrale. Le transducteur 11 pourrait aussi être, par exemple, en forme de secteur de disque, ou pourvu d'une légère incurvation, et alors en forme de calotte ou de dôme. Il a une direction d'extension principale, selon sa plus grande dimension (direction inscrite dans le plan quand le transducteur 11 est plan, ou diamétrale dans le cas d'un disque) parallèle ou essentiellement parallèle à l'axe du manchon X. La périphérie du solide 12 comprend une deuxième partie appelée rehausse 13, bien visible à la figure 4, et qui consiste en des languettes s'étendant sur les secteurs angulaires d'un cylindre, mais séparées les unes des autres par des fentes 14. La rehausse 13 est perpendiculaire à la première partie 35 et à l'axe X du manchon 16, et elle porte un rebord ou un fraisage 15 au périmètre intérieur de sa bordure libre, opposée au solide 12. Le transducteur 11 est introduit dans le rebord ou le fraisage 15 et possède un rayon un peu plus grand, ce qui fait qu'il écarte légèrement la rehausse 13 et qu'il est retenu en elle en formant un encastrement avec précontrainte. Le transducteur 11 pourrait aussi être au contact de la rehausse 13 entre la bordure libre et la première partie 35.

La pompe est complétée par le manchon 16, dans lequel s'écoule le fluide à pomper. Il s'agira souvent d'un liquide, mais d'autres fluides ne sont pas exclus. Le manchon 16 est souple et s'étend entre le transducteur 11 et le solide 12, en étant comprimé entre leurs deux faces en regard qui sont des surfaces d'appui, respectivement 17 et 18. Il comporte deux raccords 19 et 20 opposés traversant la rehausse 13 et qui finissent en saillie vers l'extérieur. On peut leur raccorder des cathéters, qui prolongeront donc le manchon 16. Les raccords 19 et 20 ont une section plus petite que celle du reste du manchon 16, qui forme donc un petit réservoir intermédiaire du liquide. Ainsi, la rehausse 13 comporte des ouvertures 36 et 37, à travers lesquelles s'étend le manchon 16.

Ainsi, le manchon 16 s'étend dans une cavité 38 délimitée par la face 17 du transducteur, la surface d'appui 18 du solide 12, et la surface interne de la rehausse 13. Le manchon peut alors être comprimé entre la surface d'appui interne 17 du transducteur, qui joue ici le rôle de butée, et la surface d'appui 18 du solide, qui subit un mouvement d'oscillation sous l'effet de l'actionnement du transducteur 11.

La réalisation des figures 4 et 5 diffère quelque peu de celle de la figure 3, parce que le manchon 16 n'est pas comprimé entre le solide 12 et le transducteur 11, mais entre le solide 12 et une plaque intermédiaire 21, introduite dans la rehausse 13, sous le transducteur 11, en étant également encastrée dans la rehausse 13. Le dispositif n'est par ailleurs pas modifié, ni son fonctionnement. Une cavité 22 s'étend entre la plaque 21 et le transducteur 11, qu'il est possible d'occuper par une carte électronique, par exemple.

Un bouchon 24 est placé sur la pompe 10 pour couvrir le transducteur 11, et il possède un rebord externe 25, qui s'ajuste autour du bord libre de la rehausse 13, de manière à la contracter, quand le bouchon 24 est enfoncé, ce qui maintient ou renforce l'encastrement du transducteur 11. Le bouchon 24 peut être inséré de force à la main ou avec une presse ou bien vissé si un filetage a été réalisé pour transmettre via la base de la rehausse 13 où est encastré le transducteur 11 un complément réglable de précontrainte radiale sur le transducteur.

Le fonctionnement du dispositif est le suivant. Une excitation appliquée au transducteur 11 produit sur lui des contractions et extensions alternées selon les flèches doubles K de la figure 3. Ainsi, le transducteur se déforme selon une direction parallèle à celle selon laquelle s'étend le manchon 18. Ces déformations produisent des basculements L sur les languettes de la rehausse 13. Au niveau de l'interface entre la rehausse 13 et l'anneau 12, ces basculements se produisent de part et d'autre d'un axe perpendiculaire à l'axe selon lequel s'étend le manchon 18. Ainsi, la rehausse 13 se comporte comme un balancier, oscillant de part et d'autre de la direction selon laquelle elle s'étend. Il en résulte des oscillations M de flexion de l'anneau 12 perpendiculaires à l'axe X parallèle à l'axe du manchon 18, ces oscillations pouvant être amplifiées au centre à cause de la réduction d'épaisseur et de l'évidement central. Les oscillations M du solide 12 produisent des battements du manchon 16 par lesquels sa section est successivement contractée puis relâchée, ce qui suscite l'écoulement du fluide à travers lui, d'un des raccords 19 à l'autre des raccords 20.

On va maintenant revenir sur certaines particularités et certains avantages de la pompe conforme à l'invention.

La direction de l'écoulement, qui est la direction de l'axe X selon lequel s'étend le manchon 16, d'un raccord 19 à l'autre 20, est ici parallèle au plan d'extension du solide 12 et du transducteur 11. Il en résulte que l'encombrement de la pompe, du moins dans la direction perpendiculaire à l'axe X, peut être très réduit. Cela est particulièrement avantageux dans certaines applications comme un implant chirurgical, où une pompe très plate est souhaitée, tout en pouvant sans inconvénient posséder d'autres dimensions plus importantes. La pompe de l'invention peut ainsi être munie d'un résonateur 10 de grand rayon tout en satisfaisant au critère de faible dimension de hauteur, ce qui signifie que le transducteur 11 et le solide 12 peuvent avoir des dimensions suffisantes pour communiquer une énergie de pompage notable.

Dans la réalisation des figures 1 et 2, il est beaucoup plus difficile de réduire l'une ou l'autre des dimensions de la pompe, puisque le manchon 3 doit avoir une longueur suffisante pour être soumis à des oscillations d'amplitude aptes à permettre le pompage, et, d'autre part, le rayon du résonateur 2 doit rester suffisant.

L'encastrement du transducteur 11 dans la rehausse 13 permet d'éviter tout collage du transducteur 11, et donc un filtrage des vibrations à travers la couche de colle, et les risques éventuels de mauvaise fabrication ou de décollage futur. On comprend alors qu'un encastrement du transducteur est préférable à un simple collage.

La séparation physique du transducteur 11 et du solide 12 permet de les construire chacun avec une liberté beaucoup plus grande, en ce qui concerne notamment leurs dimensions.

L'action du solide 12 sur le manchon 16 s'effectue par des contractions de sa section plutôt que par des cisaillements, comme dans la réalisation précédente, ce qui devrait assurer un pompage plus efficace. Le contact entre le manchon 16 et le solide 12 par toute une face de plus grande superficie de la première partie 35 (c'est-à-dire la surface d'appui 18 de celui-ci) devrait avoir le même effet, le manchon 16 étant placé dans la cavité 38.

Le dispositif précédent nécessitait l'utilisation de modes particuliers de vibrations de l'anneau 70, afin de produire la flexion du manchon 3 nécessaire. L'invention permet d'obtenir les contractions du manchon 16 au moyen de nombreux modes de vibrations différents du solide 12 en forme de disque, ce qui permet d'obtenir des caractéristiques différentes d'injection du fluide, par exemple sous forme de goutte à goutte en utilisant des modes à basse fréquence, ou sous forme de débit continu, en utilisant des modes à fréquences plus élevées.

La constitution du manchon 16, avec une section renflée entre les raccords 19 et 20, ou les ouvertures 36 et 37 ménagées dans la rehausse 13, permet de constituer un petit réservoir intermédiaire inscrit dans la rehausse 13. Par petit, on entend un volume compris entre 1µl et 50 µl voire 100 µl. On obtient alors des caractéristiques de pompage plus uniformes que dans la réalisation antérieure, puisque le pompage dépend moins de la pression à l'intérieur du réservoir amont et de son degré de remplissage. Et la rehausse 13 entourant le manchon 16 le retient sans moyen de fixation. La pompe forme un module complètement autonome. Autrement dit, le manchon est retenu dans la cavité du fait que le diamètre du réservoir intermédiaire est supérieur au diamètre des ouvertures 36 et 37 pratiquées dans la rehausse 13.

Le manchon se prête aussi à des écoulements réversibles, puisque certains modes de vibration du solide 12 peuvent favoriser la circulation du fluide dans un sens ou dans l'autre. Même sans recourir à cette propriété, la disposition symétrique de la pompe entre les raccords 19 et 20 se prête à des inversions de sens de circulation, en utilisant, par exemple, des valves anti-retour, des capillaires fins ou des embouts spécifiques, d'un côté ou de l'autre de la pompe. Le sens de l'écoulement peut être imposé par une valve unidirectionnelle indépendante de l'invention placée d'un côté ou de l'autre des embouts 19 ou 20 du manchon 16.

Un dispositif d'injection complet peut être celui qui est schématisé à la figure 6. La ligne d'injection comprend un cathéter amont 26 et un cathéter aval 27 des deux côtés de la pompe 20, respectivement joints aux raccords 19 et 20 du manchon 16. Le cathéter amont 26 s'étend jusqu'à un réservoir 28 de fluide à injecter, et le cathéter aval 27 débouche dans un lieu d'injection 29, appartenant, par exemple, à un être vivant. Un excitateur 30 comprenant un circuit de commande excite le transducteur 11. L'injection du fluide est alors amorcée, et elle cesse dès que la fourniture d'électricité s'interrompt au transducteur 11. Une valve 31, prévue pour s'ouvrir sous une surpression déterminée, est ajoutée sur le cathéter aval 27 pour empêcher toute fuite au repos si les cathéters 26 et 27 sont trop larges pour arrêter l'écoulement.

## Revendications

1. Pompe d'injection d'un fluide, comprenant un résonateur et un manchon, le résonateur (10) comprenant un transducteur (11) piézoélectrique et un solide (12) que le transducteur déforme, et le manchon (16) étant parcouru par le fluide pendant l'injection et s'étendant selon un axe (X), le manchon étant apte à être déformé par le solide se déformant, **caractérisée en ce que** le résonateur comprend une surface d'appui (17), destinée à entrer en contact avec le manchon (16) lorsque le manchon (16) est déformé par ledit solide, et le transducteur (11) s'étend parallèlement à l'axe du manchon.

2. Pompe d'injection suivant la revendication 1, **caractérisé en ce que** le solide (12) comprend une première partie (35) s'étendant dans une direction essentiellement parallèle à l'axe (X) du manchon et comprenant une surface (18) par laquelle le manchon est déformé.

3. Pompe d'injection suivant la revendication 2, **caractérisée en ce que** le solide comprend une deuxième partie (13) solidaire de la première partie et s'étendant selon une direction transversale, pouvant être perpendiculaire, à l'axe du manchon.

4. Pompe d'injection suivant la revendication 3, **caractérisée en ce que** ladite deuxième partie forme une rehausse (13), qui s'étend à partir d'une zone périphérique de ladite première partie, qui est annulaire.

5. Pompe d'injection suivant la revendication 3 ou 4, **caractérisée en ce que** ladite deuxième partie (13) est en contact avec le transducteur (11) pour le maintenir.

6. Pompe d'injection suivant les revendications 4 et 5, **caractérisée en ce que** le transducteur (11) est précontraint et comprimé dans ladite deuxième partie (13).

7. Pompe d'injection suivant l'une quelconque des revendications 5 ou 6, **caractérisée en ce que** le contact entre le transducteur (11) et ladite deuxième partie (13) s'effectue soit à un bord libre de la deuxième partie (13), soit entre ledit bord libre et ladite première partie (35).

8. Pompe d'injection suivant l'une quelconque des revendications 3 à 7, **caractérisée en ce que** la première partie et la deuxième partie du solide (12) délimitent une cavité (38) à travers laquelle s'étend le manchon.

9. Pompe d'injection suivant la revendication 8, **caractérisée en ce que** la seconde partie comprend des ouvertures (36, 37) que traverse le manchon.

10. Pompe d'injection suivant l'une quelconque des revendications 2 à 9, **caractérisée en ce que** la première partie du solide (12) est amincie, voire évidée, à une portion centrale.

11. Pompe d'injection suivant l'une quelconque des revendications 2 à 10, **caractérisée en ce que** la première partie (35) du solide (12) est agencée dans le résonateur (10) pour subir une oscillation en direction perpendiculaire à l'axe (X) du manchon.

12. Pompe d'injection suivant l'une quelconque des revendications 1 à 11, **caractérisée en ce que** le transducteur (11) est soit plan soit incurvé, notamment en forme de disque ou de secteur de disque.

13. Pompe d'injection suivant l'une quelconque des revendications 1 à 12, **caractérisée en ce que** le manchon est comprimé entre ladite surface d'appui (17), qui appartient soit au transducteur (11), soit à une plaque (21) intermédiaire au solide et au transducteur, et une deuxième surface (18) qui appartient au solide (12).

14. Pompe d'injection suivant l'une quelconque des revendications 3 à 9, **caractérisée par** un bouchon (24) ajusté sur un bord libre, opposé à la première partie, de la deuxième partie.

15. Dispositif d'injection de fluide, comprenant un réservoir (28) du fluide, deux cathéters (26, 27) reliant le réservoir à un lieu d'injection (29) du fluide, **caractérisé en ce qu'**il comprend une pompe d'injection conforme à l'une quelconque des revendications précédentes, dont le manchon (16) relie les cathéters entre eux, et un excitateur (30) du transducteur (1).

16. Dispositif d'injection de fluide suivant la revendication 15, **caractérisé en ce qu'**un des cathéters, adjacent au point d'injection du fluide, est muni d'une valve (31) d'ouverture à une pression déterminée.

## Patentansprüche

1. Fluidinjektionspumpe, umfassend einen Resonator und eine Muffe, wobei der Resonator (10) einen piezoelektrischen Wandler (11) und einen Festkörper (12) umfasst, den der Wandler verformt, und wobei die Muffe (16) während der Injektion von dem Fluid durchströmt wird und sich entlang einer Achse (X) erstreckt, wobei die Muffe dazu ausgelegt ist, durch den sich verformenden Festkörper verformt zu werden, **dadurch gekennzeichnet, dass** der Resonator eine Anlageoberfläche (17) umfasst, die dazu ausgelegt ist, in Kontakt mit der Muffe (16) zu gelangen, wenn die Muffe (16) von dem Festkörper verformt wird, und sich der Wandler (11) parallel zur Achse der Muffe erstreckt.

2. Injektionspumpe nach Anspruch 1, **dadurch gekennzeichnet, dass** der Festkörper (12) einen ersten Teil (35) umfasst, der sich in einer Richtung im Wesentlichen parallel zur Achse (X) der Muffe erstreckt und eine Oberfläche (18) umfasst, durch die die Muffe verformt wird.

3. Injektionspumpe nach Anspruch 2, **dadurch gekennzeichnet, dass** der Festkörper einen zweiten Teil (13) umfasst, der mit dem ersten Teil verbunden ist, und sich entlang einer Querrichtung erstreckt, die orthogonal zur Achse der Muffe sein kann.

4. Injektionspumpe nach Anspruch 3, **dadurch gekennzeichnet, dass** der zweite Teil einen Aufsatz (13) bildet, der sich ausgehend von einer Umfangszone des ersten Teils erstreckt, der ringförmig ist.

5. Injektionspumpe nach Anspruch 3 oder 4, **dadurch gekennzeichnet, dass** der zweite Teil (13) in Kontakt mit dem Wandler (11) ist, um ihn zu halten.

6. Injektionspumpe nach den Ansprüchen 4 und 5, **dadurch gekennzeichnet, dass** der Wandler (11) in dem zweiten Teil (13) vorgespannt und komprimiert ist.

7. Injektionspumpe nach einem der Ansprüche 5 oder 6, **dadurch gekennzeichnet, dass** der Kontakt zwischen dem Wandler (11) und dem zweiten Teil (13) entweder an einem freien Rand des zweiten Teils (13) oder zwischen dem freien Rand und dem ersten Teil (35) erfolgt.

8. Injektionspumpe nach einem der Ansprüche 3 bis 7, **dadurch gekennzeichnet, dass** der erste Teil und der zweite Teil des Festkörpers (12) einen Hohlraum (38) begrenzen, durch den sich die Muffe hindurch erstreckt.

9. Injektionspumpe nach Anspruch 8, **dadurch gekennzeichnet, dass** der zweite Teil Öffnungen (36, 37) umfasst, die die Muffe durchquert.

10. Injektionspumpe nach einem der Ansprüche 2 bis 9, **dadurch gekennzeichnet, dass** der erste Teil des Festkörpers (12) in einem zentralen Bereich abgedünnt oder ausgehöhlt ist.

11. Injektionspumpe nach einem der Ansprüche 2 bis 10, **dadurch gekennzeichnet, dass** der erste Teil (35) des Festkörpers (12) in dem Resonator (10) angeordnet ist, um eine Oszillation in einer Richtung orthogonal zur Achse (X) der Muffe zu erfahren.

12. Injektionspumpe nach einem der Ansprüche 1 bis 11, **dadurch gekennzeichnet, dass** der Wandler (11) entweder flach oder gekrümmt ist, insbesondere in Form einer Scheibe oder eines Scheibensektors.

13. Injektionspumpe nach einem der Ansprüche 1 bis 12, **dadurch gekennzeichnet, dass** die Muffe komprimiert ist zwischen der Anlageoberfläche (17) die entweder zum Wandler (11) gehört, oder zu einer Scheibe (21) zwischen dem Festkörper und dem Wandler, und einer zweiten Oberfläche (18), die zum Festkörper (12) gehört.

14. Injektionspumpe nach einem der Ansprüche 3 bis 9, **gekennzeichnet durch** einen Stopfen (24), der an einen freien Rand des zweiten Teils entgegengesetzt zum ersten Teil angepasst ist.

15. Fluidinjektionsvorrichtung umfassend einen Fluidbehälter (28) und zwei Katheter (26, 27), die den Behälter mit einer Fluidinjektionsstelle (29) verbinden, **dadurch gekennzeichnet, dass** sie eine Injektionspumpe gemäß einem der vorhergehenden Ansprüche umfasst, deren Muffe (16) die Katheter miteinander verbindet, sowie einen Erreger (30) für den Wandler (1).

16. Fluidinjektionsvorrichtung nach Anspruch 15, **dadurch gekennzeichnet, dass** einer der Katheter, der der Fluidinjektionsstelle benachbart ist, mit einem Ventil (31) zum Öffnen bei einem vorbestimmten Druck ausgestattet ist.

## Claims

1. Fluid injection pump for a fluid, comprising a resonator and a sleeve, the resonator (10) comprising a piezoelectric transducer (11) and a solid which the transducer deforms, and the sleeve (16) being flown by the fluid during the injection and extending along an axis (X), the sleeve being deformable by the solid when the latter deforms, **characterised in that** the resonator comprises a support surface (17) for contacting the sleeve (16) when the sleeve (16) is deformed by the said solid, and the transducer (11) extends parallel to the axis of the sleeve.

2. Fluid injection pump according to claim 1, **characterised in that** the solid (12) comprises a first part (35) extending in a direction essentially parallel to the axis (X) of the sleeve and comprising a surface (18) by which the sleeve is deformed.

3. Fluid injection pump according to claim 2, **characterised in that** the solid comprises a second part (13) secured to the first part and extending in a transverse direction, which may be perpendicular, to the axis of the sleeve.

4. Fluid injection pump according to claim 3, **characterised in that** said second part constitutes a raising (13), which extends from a peripheral zone of said first part, which is ring-shaped.

5. Fluid injection pump according to claim 3 or 4, **characterised in that** said second part (13) contacts the transducer (11) to sustain it.

6. Fluid injection pump according to claims 4 and 5, **characterised in that** the transducer (11) is prestressed and compressed in said second part (23).

7. Fluid injection pump according to any of claim 5 or 6, **characterised in that** the contact between the transducer (11) and said second part (13) is made either at a free edge of the second part (13) or between said edge and said first part (35).

8. Fluid injection pump according to any of claims 3 to 7, **characterised in that** the first part and the second part of the solid (12) define a hollow (38) through which the sleeve extends.

9. Fluid injection pump according to claim 8, **characterised in that** the second part comprises openings (36, 37) which the sleeve passes through.

10. Fluid injection pump according to any of claims 2 to 9, **characterised in that** the first part of the solid (12) is narrower, possibly hollowed out, at a central portion.

11. Fluid injection pump according to any of claims 2 to 10, **characterised in that** the first part (35) of the solid (12) is arranged in the resonator (10) for being imparted an oscillation in a direction perpendicular to the avis (X) of the sleeve.

12. Fluid injection pump according to any of claims 1 to 11, **characterised in that** the transducer (11) is either plane or curved, especially disc-shaped or shaped as a disc sector.

13. Fluid injection pump according to any of claims 1 to 12, **characterised in that** the sleeve is compressed between said support surface (17), which belongs either to the transducer (11) or to a plate (21) intermediate the solid and the transducer, and a second surface (18) which belongs to the solid (22).

14. Fluid injection pump according to any of claims 3 to 9, **characterised by** a plug (24) fitted on a free edge, opposite the first part, of the second part.

15. Fluid injection device, comprising a tank (28) of the fluid, two catheters (26, 27) connecting the tank to an injection plate (29) of the fluid, **characterised in that** it comprises an injection pump according to any of the previous claims, the sleeve (16) of which connects the catheters together, and an exciter (30) of the transducer (1).

16. Fluid injection device according to claim 15, charaterised in that one of the catheters, adjacent to the fluid injection place, is equiped with a valve (31) opening at a predetermined pressure.
